# EUROPÄISCHE PATENTANMELDUNG

(11) **EP 0 649 901 A1**
(43) Veröffentlichungstag der Anmeldung: **26.04.1995**
(21) Anmeldenummer: 94250251.9
(22) Anmeldetag: 14.10.1994
(51) Int. Cl.: C12N 5/08, C12Q 1/02

(54) **Humane Zellinien mit Langerhans-Zell-Charakteristik**

(30) Priorität: 26.10.1993 DE 4337396
(71) Anmelder: Beiersdorf Aktiengesellschaft, D-20253 Hamburg (DE)
(72) Erfinder: Braathen, Lasse R., CH-3006 Bern (CH); Nunez, Rafael, CH-3123 Belp (CH)
(74) Vertreter: UEXKÜLL & STOLBERG

(57) **Zusammenfassung**

Die Erfindung betrifft permanente Zellinien myeloiden Ursprungs, die funktionelle und antigene Eigenschaften von Langerhans-Zellen aufweisen. Die in vitro stabil und ohne Funktionsverlust unbegrenzt kultivierbaren Zellinien eignen sich in vorteilhafter Weise zur Durchführung von biologischen, immunologischen, medizinischen, pharmakologischen, toxikologischen und/oder kosmetischen Untersuchungen.

## Beschreibung

Die vorliegende Erfindung betrifft permanente Zellinien, die funktionelle und antigene Eigenschaften von Langerhans-Zellen aufweisen und in vitro ohne Funktionsverlust kultivierbar sind, sowie Verfahren zur Herstellung dieser Zellinien und deren Verwendung für biologische, immunologische, medizinische, pharmakologische, toxikologische und/oder kosmetische Untersuchungen. Darüber hinaus betrifft die Erfindung ein Diagnose-Kit für die genannten Untersuchungen, das eine oder mehrere dieser permanenten Zellinien enthält.

Im Rahmen der körpereigenen Abwehr spielt das Haut-Immunsystem bekanntermaßen eine besondere Rolle, da die Haut ein immunologisch aktives und in seiner zellulären Zusammensetzung besonderes Organ darstellt. Langerhans-Zellen bilden einen der wesentlichen zellulären Bestandteile dieses Immunsystems (Immunology Today 14, (2) 75 (1993)), und sie stellen 1 bis 2% der gesamten epidermalen Zellpopulation. Als antigen-präsentierende Zellen üben die zur Gruppe der dendritischen Zellen gehörenden Langerhans-Zellen zentrale Funktionen aus, da sie mit hoher Effizienz Antigene und Allergene präsentieren (Virch. Arch. A. (Pathol. Anat.) 44, 1868, 325). Als eine der Hauptfolgen werden dabei T-Lymphozyten aktiviert, was beispielsweise zur Ausbildung von allergischen Reaktionen führen kann. Neben ihrer dendritischen Morphologie werden Langerhans-Zellen durch die Expression spezifischer Oberflächen-Moleküle (z.B. CD1a, HLA-DR, CD11a) charakterisiert (Lab. Invest. 45, 465 (1981)). Bei Kontakt mit Antigenen oder Allergenen sezernieren Langerhans-Zellen ein breites Spektrum von Zytokinen, die immunologische Folgereaktionen hervorrufen (Immunology 89, 1398 (1992); J. Invest. Dermatol. 99, 537 (1992)). Darüber hinaus sind sie an der Entstehung und am Verlauf verschiedener Erkrankungen beteiligt, wie z.B. bei der Einnistung von HIV-Viren bei AIDS (J. Invest. Dermatol. 88, 233 (1987)).

Langerhans-Zellen spielen ferner beispielsweise eine große Rolle bei der Ätiopathogenese, bei toxisch-irritativen und allergischen Kontaktekzemen, bei atopischer Dermatitis sowie bei der Krebsbekämpfung.

Um die Beteiligung von Langerhans-Zellen an der Entstehung von Allergien vom verzögerten Typ (J. Invest. Dermatol. 82, 440 (1984)), die Erhöhung der systemischen Immunität durch Vorbehandlung mit Zytokinen (J. Immunol. 140, 1457 (1987)) sowie die Rolle der Langerhans-Zellen bei immunkompetenten zellulären Abwehrmechanismen (Nature 282, 326 (1979)) besser verstehen zu können, hat man im Stand der Technik versucht, Langerhans-Zellen für in vitro-Untersuchungen zu isolieren und zu kultivieren.

Durch Verwendung einer Saugvorrichtung lassen sich z.B. bei Probanden auf der Haut Blasen erzeugen (Scand. J. Immunol. II, 401 (1980)), deren Dach ausschließlich aus Epidermis besteht. Trägt man diese Epidermisschicht ab und behandelt sie mit einer aus Trypsin und DNAse bestehenden Enzymmischung, so kann nach einem langwierigen Prozeß eine Einzelzellsuspension erhalten werden, die aus einigen Millionen epidermaler Zellen besteht, wobei Langerhans-Zellen nur mit einem Anteil von etwa 2% vorliegen. Mit Hilfe verschiedener zusätzlicher, aufwendiger Isolationstechniken ist es möglich, Langerhans-Zellen in den Suspensionen anzureichern (Scand. J. Immunol. 19, 255 (1984)) und die gewonnenen Zellen auf ihr funktionelles Verhalten hin zu überprüfen.

Ein weiteres, im Stand der Technik bekanntes Verfahren basiert auf der chirurgischen Entfernung von Haut, wobei die oberste Hautschicht abgetrennt und ebenfalls mit Enzymen behandelt wird. Diese Technik, die auch zum Ziel hat, eine Einzelzellsuspension zu erhalten, ist jedoch im Hinblick auf die Vitalität und Ausbeute von Langerhans-Zellen weniger brauchbar.

Nach einem neueren Verfahren können Langerhans-Zellen aus periphärem Blut gewonnen werden (Immunology Letters 31, 189 (1992)). Nach diesem ebenfalls sehr aufwendigen und zeitintensiven Verfahren können nur äußerst begrenzte Zellzahlen erhalten werden, die in Kultur nur eine sehr geringe Stabilität aufweisen.

Neben der schwierigen, meist aufwendigen Isolierung aus Blut oder Körpergewebe sowie dem geringen Anteil (2%) von Langerhans-Zellen im Gewebe steht der rasche Funktionsverlust von Langerhans-Zellen bereits kurze Zeit nach der Isolierung (J. Invest. Dermatol. 99, 237 (1992)) einer umfassenden Verwendung von Langerhans-Zellen in vitro eindeutig entgegen.

Zahlreiche Versuche, Langerhans-Zellinien zu erzeugen, blieben bislang ohne Erfolg. Bei Versuchen mit epidermalen Zellkulturen, die Langerhans-Zellen mit einem Anteil von 2% enthalten oder Kulturen mit hochgereinigten Langerhans-Zellen verschwinden die HLA-DR- und CD1a-Oberflächenmarker im Verlauf von 5 bis 6 Tagen (J. Exp. Med. 166, 1484 (1987)). Außerdem konnte beobachtet werden, daß die antigen-präsentierende Eigenschaft der Zellen, die in vivo hauptsächlich zur Aktivierung von T-Lymphozyten führt, ebenfalls verlorengeht. Dieser Aktivitäts- bzw. Funktionsverlust beruht aller Wahrscheinlichkeit nach auf einem Absterben der Zellen in Kultur.

Versuche, isolierte, humane Langerhans-Zellen für die Durchführung immunologischer Untersuchungen über einen längeren Zeitraum am Leben zu erhalten, blieben daher bisher ohne Erfolg. Außerdem waren angereicherte Langerhans-Zellisolate bislang stets mit einer relativ großen Beimischung Keratinozyten verunreinigt, die die Aussagekraft der mit Hilfe dieser Zellen erhaltenen Resultate stark beeinträchtigen. Die Verwendung von Langerhans-Zellen für in vitro-Routineuntersuchungen im biologischen, medizinischen, pharmakologischen oder toxikologischen Bereich war im Stand der Technik daher bislang nicht möglich.

Aufgabe der Erfindung ist es daher, Zellinien zu schaffen, die funktionelle und antigene Eigenschaften von Langerhans-Zellen aufweisen und die in vitro stabil und ohne Funktionsverlust unbegrenzt kultivierbar sind.

Erfindungsgemäß wird diese Aufgabe durch permanente Zellinien myeloiden Ursprungs gelöst, die funktionelle und antigene Eigenschaften von Langerhans-Zellen aufweisen, d.h. die Zellinien exprimieren die klassischen Langerhanszell-Oberflächenantigene CD1a und HLA-DR und sind darüberhinaus in der Lage, Antigene zu präsentieren.

Die erfindungsgemäßen Zellinien sind in besonders bevorzugter Weise von Monozyten oder deren Vorläufern abgeleitet.

Es wurde überraschenderweise gefunden, daß aus myeloiden Linien abgeleitete stabile Zellinien funktionelle und antigene Charakteristika von Langerhans-Zellen aufweisen, obwohl diese bei den Ausgangszellinien nicht nachgewiesen werden konnten.

Gemäß einer Ausführungsform der Erfindung stammen die permanenten Zellinien von der Monozyten-Myelom-Zellinie U-937 ab, die ursprünglich aus einem histiozytischen Lymphom, d.h. einer Krebszellinie, isoliert wurde und keine Langerhans-Charakteristika aufweist (Int. J. Cancer 17, 565-577 (1976)). Neben U-937 lassen sich auch andere Myelom-Zellinien, wie z.B. HL-60, zur Herstellung der erfindungsgemäßen permanenten Zellinien mit Langerhans-Zell-Charakteristik einsetzen.

Im Rahmen der vorliegenden Erfindung wurden aus der Monozyten-Myelom-Zellinie U-937 die permanenten Zellinien RAN1 und LAS1 abgeleitet, die am 29. Juli 1993 bei der Deutschen Sammlung von Mikroorganismen und Zellkulturen GmbH (DSM), Mascheroder Weg 1b, 31824 Braunschweig hinterlegt wurden:
- LAS1:: Hinterlegungsnummer DSM ACC2139
- RAN1:: Hinterlegungsnummer DSM ACC2140
Die Zellinien RAN1 und LAS1 wachsen im Gegensatz zu der Ausgangszellinie U-937 in flüssigem Kulturmedium in Form flacher Aggregate oder Kolonien, wobei jede Zelle fest mit ihren Nachbarzellen und dem Plastiksubstrat verbunden ist. Während die U-937-Zellen eine variable Morphologie und Größe aufweisen, sind die Zellen der Linien RAN1 und LAS1 hinsichtlich dieser Eigenschaften homogen. Bei einer Kultivierung in festem Medium erhalten die erfindungsgemäß bevorzugten Zellinien RAN1 und LAS1 eine dendritische Morphologie mit mehr als 4 dendritischen Cytoplasma-Ausläufern, die nach 48-stündiger Kulturdauer in Kontakt mit den Nachbarzellen treten, was auf einen positiven Tropismus und Motilität hinweist.

Es wurde überraschenderweise festgestellt, daß im Gegensatz zur Ausgangszellinie U-937, die sich sowohl morphologisch als auch hinsichtlich des funktionellen und antigenen Charakters deutlich von Langerhans-Zellen unterscheidet, die erfindungsgemäßen Zellinien, vorzugsweise RAN1 und LAS1, überaschenderweise Oberflächenantigene exprimieren, die denjenigen von Langerhans-Zellen entsprechen und die bei der Monozyten-Myelom-Zellinie U-937 zuvor nicht nachgewiesen werden konnten. Auch entsprechen die erhaltenen Zellinien hinsichtlich ihrer Funktionalität Langerhans-Zellen, obwohl diese Charakteristik bei den Mylom-Zellen zuvor nicht beobachtet wurde.

In Tabelle 1 sind Oberflächenantigene der Monozyten-Myelom-Zellinie U-937 sowie der daraus abgeleiteten erfindungsgemäßen Zellinien RAN1 und LAS1 mit den bei Langerhans-Zellen identifizierten Oberflächenantigenen verglichen.

**Tabelle 1**

| | CD1a | HLA-DR | CD11a | CD14 | CD4 | CD8 | CD54 | CD45 | CD23 |
|---|---|---|---|---|---|---|---|---|---|
| U937 | - | + | - | - | + | - | ++ | ++ | + |
| RAN1 | ++ | ++ | ++ | - | - | - | - | - | + |
| LAS1 | ++ | ++ | ++ | - | - | - | - | - | + |
| LZ | ++ | ++ | ++ | - | - | - | - | - | + |
| LZ = Langerhans-Zellen + = positiver Nachweis / - = nicht nachgewiesen | | | | | | | | | |

Gemäß einer weiteren Ausführungsform der Erfindung können die permanenten Zellinien von den Zellinien RAN1 oder LAS1 abgeleitet werden, wobei die Zellinien RAN1 bzw. LAS1 durch chemische, thermische, mechanische, licht-vermittelte, zell- und/oder mikrobiologische Behandlungsverfahren verändert werden können. Vorzugsweise umfassen bzw. beinhalten solche Behandlungsverfahren die Einwirkung eines oder mehrerer biologischer Mediatorstoffe, insbesondere von Zytokinen und/oder Interleukinen etc., die alleine oder in Kombination mit einem der vorstehenden Behandlungsverfahren von RAN1 oder LAS1 eingesetzt werden.

Verglichen mit Langerhans-Zellen weisen die erfindungsgemäßen permanenten Zellinien, die vorzugsweise humanen Ursprungs sind, eindeutig funktionelle und antigene Eigenschaften von Langerhans-Zellen auf, sie können aber im Gegensatz zu diesen in vitro ohne Funktionsverluste kultiviert werden. Die Zellinien der vorliegenden Erfindung sind dadurch gekennzeichnet, daß sie eines, mehrere oder alle der Oberflächenantigene CD1a, HLA-DR, CD11a und CD23 exprimieren, die auch auf Langerhans-Zellen vorhanden sind. Darüber hinaus handelt es sich bei den neuen, kultivierbaren Zellinien ebenfalls um antigen-präsentierende Zellen, die eine Aktivierung vorher nicht-aktivierter T-Lymphozyten auslösen können, d.h. sie verfügen wie normale Langerhans-Zellen ebenfalls nicht nur über antigen-präsentierende Strukturen, sondern auch über die zur Auslösung einer T-Zellantwort auf ein Antigen notwendigen costimulierenden Faktoren. Die erfindungsgemäßen permanenten Zellinien, die eine Serie von Zytokinen sezernieren können, sind in Kultur im Gegensatz zu normalen Langerhans-Zellen ohne Funktionsverlust kultivierbar. In Analogie zu Langerhans-Zellen kann die Fähigkeit zur Antigen-Repräsentation durch UV-B-Licht unterdrückt werden.

Ein spezifisches Merkmal von Langerhans-Zellen ist deren Beteiligung an der Entstehung von Allergien des verzögerten Typs (Typ IV, Delayed Type Hypersensitivity). Zur Induktion einer Allergie ist die entsprechende Reaktionskaskade der Langerhans-Zellen essentiell. Neueste Untersuchungen (J. Immunol. 150 (9), 3698 (1993)) weisen darauf hin, daß die Entscheidung über das allergene Potential eines Stoffes auf der Ebene der Langerhans-Zelle in der ersten halben Stunde des Erstkontaktes des Allergens mit den Langerhans-Zellen fällt. Das Potential des Allergens ist an dem in den Langerhans-Zellen aktivierten Genmuster erkennbar. Diese für Langerhans-Zellen charakteristische Zellaktivierung ist auch bei den erfindungsgemäßen Zellinien möglich, z.B. wenn sie mit dem bekannten Allergen Urushiol in Kontakt gebracht werden. Die permanenten Zellinien reagieren wie normale Langerhans-Zellinien spezifisch mit einer schnellen IL1β-Genaktivierung, die bei nicht-allergenen sondern irritant wirkenden Prüfsubstanzen nicht stattfindet. Die Zellinien werden dabei mit den Prüfsubstanzen über einen Zeitraum von bis zu 30 Minuten inkubiert, wodurch es zur Induktion von IL1β-mRNA kommt.

Durch diese normalen Langerhans-Zellen entsprechenden funktionellen Charakteristika ermöglichen die erfindungsgemäßen Zellinien, vorzugsweise RAN1 und LAS1, eine Analyse des allergischen Potentials verschiedener Prüfsubstanzen, d.h. z.B. von Einzelstoffen oder komplexen Stoffmischungen.

Die Langerhans-Zellen der Haut bilden die vorderste Linie der immunkompetenten zellulären Abwehrmechanismen des Immunsystems gegenüber durch die Haut eindringenden Mikroorganismen. Wie im Stand der Technik gezeigt wurde (Immunology Today 14 (8), 383 (1993)), gilt dies sowohl für parasitäre Erkrankungen wie der Leishmaniase durch *Leishmania*-Spezies als auch für bakterielle Erkrankungen wie Lepra, die durch *Mycobacterium leprae* ausgelöst wird. Bei beiden Krankheiten stehen bei persistierenden Formen eine unvollständige Abtötung der u.a. durch Langerhans-Zellen aufgenommenen Mikroorganismen sowie eine nicht-effiziente Präsentation der Antigene der Mikroorganismen einer Auslösung der T-Zell-vermittelten Immunantwort entgegen. Die Effizienz der Abwehrzellen kann im Rahmen einer erhöhten systemischen Immunität durch Vorbehandlung mit Zytokinen, wie z.B. Tumornekrosefaktor α (TNFα), verbessert werden. Dabei kommt es u.a. zu einer verstärkten Expression antigen-präsentierender Moleküle auf den relevanten Immunzellen und damit zu einer effektiveren Präsentation.

Anhand dieses Phänomens, das auch bei den erfindungsgemäßen permanenten Zellinien, insbesondere RAN1 und LAS1, nachgewiesen werden konnte, läßt sich darüber hinaus außerdem untersuchen, in welchem Umfang Immunzellen in vivo auf eine Infektion reagieren können. Damit läßt sich insbesondere eine Veränderung der immunologischen Abwehr unter Einfluß von Medikamenten oder anderen Therapieformen mittels einer entsprechenden, durch die erfindungsgemäßen Zellinien induzierten T-Zell-Antwort untersuchen. Zu diesem Zweck wird die Zellinie einer entsprechenden Vorbehandlung mit dem Medikament oder der Therapieform unterzogen und dann mit den möglichst histokompatiblen, aufgereinigten T-Lymphozyten co-kultiviert. Eine durch die Vorbehandlung veränderte Funktionalität der Zellinie, die sich in einer Modifikation der T-Lymphozyten-Antwort niederschlägt, kann durch Standardverfahren hinsichtlich der T-Lymphozyten-Aktivierung quantifiziert werden. Ein weiterer experimenteller Ansatz besteht darin, daß das unter dem Einfluß von Medikamenten oder Therapieformen veränderte Reaktionsprofil von T-Lymphozyten mittels der erfindungsgemäßen Zellinien überprüft und quantifiziert werden kann. Veränderungen im Immunstatus einer untersuchten Person lassen sich mit Hilfe dieser Verfahren somit detailliert untersuchen.

Daß analog zu den normalen Langerhans-Zellen auch die erfindungsgemäßen Zellinien und insbesondere RAN1 und LAS1 in der Lage sind, vorher nicht-aktivierte T-Lymphozyten zu aktivieren, bestätigt, daß auch sie über die notwendigen costimulierenden Faktoren verfügen. Somit eignen sich die permanenten Zellinien der vorliegenden Erfindung für sämtliche Untersuchungen, die eine für Langerhans-Zellen typische Bearbeitung des Antigens betreffen, wie z.B. Prozessierung, selektive Präsentation von Bruchstücken etc.

Unter dem Einfluß von UV-Licht, insbesondere im UV-B-Bereich, kommt es in der Haut zu einer Unterdrückung allergischer Reaktionen des verspäteten Typs (Typ IV). Ursache hierfür ist die unter dem Einfluß von UV-B-Licht auftretende mangelhafte Funktion bzw. Abwesenheit von Langerhans-Zellen in der Haut. Auch in vitro ist dieser Effekt nachweisbar. Dabei können Langerhans-Zellen durch eine mittels UV-B-Licht induzierte Inaktivierung ihre Fähigkeit verlieren, Antigene zu präsentieren. Dieses für normale Langerhans-Zellen typische Phänomen kann auch bei den erfindungsgemäßen Zellinien, z.B. RAN1 und LAS1 in vitro nachgewiesen werden. Unter dem Einfluß von UV-B-Licht verlieren auch sie ihre Fähigkeit zur Antigen-Präsentation, und analog den normalen Langerhans-Zellen sind sie nicht mehr in der Lage, eine sogenannte "mixed lymphocyte reaction" auszulösen.

Bei den erfindungsgemäßen Zellinien konnte ebenfalls eine Infizierbarkeit mit HTLV-IIIb (HIV) nachgewiesen werden, wie sie für Langerhans-Zellen charakteristisch ist.

Die neuen Zellinien ermöglichen somit eine breite Anwendbarkeit im Rahmen der HIV-Forschung und Diagnostik, die im Stand der Technik mit normalen Langerhans-Zellen bislang nicht möglich war.

Vorzugsweise werden die permanenten Zellinien der vorliegenden Erfindung hergestellt, indem man a) Zellen einer myeloiden Linie auswählt, b) diese Zellen proliferieren läßt, c) an Plastik adhärente Zellen selektiert, d) eine erste limitierende Verdünnungsselektion durchführt, e) an Plastik adhärente Zellen selektiert, f) eine zweite limitierende Verdünnungsselektion durchführt, und g) an Plastik adhärente Zellen selektiert, die funktionelle und antigene Eigenschaften von Langerhans-Zellen aufweisen.

Vorzugsweise führt man das Verfahren durch, indem man in Stufe c) nacheinander die Passagen 1, 2, 4, 6, und 8 durchführt, wobei man in Passage 6 CD1⁺ Zellen und in Passage 8 Klone mit flacher Morphologie selektiert, in Stufe d) Passage 10 und in Stufe e) Passage 12 durchführt, wobei man in Passage 12 CD1⁺ Zellen selektiert, in Stufe f) Passage 14 und in Stufe g) nacheinander die Passagen 16, 18 und 20 durchführt, wobei in Passage 16 flache Klone und in Passage 18 CD1⁺ Zellen selektiert, die die oben genannten Eigenschaften aufweisen.

Die einzelnen, im Stand der Technik bekannten Schritte dieses Verfahrens (Passagen) sind an die bei der Isolierung von Keratinozyten angewendeten Vorgehensweise angelehnt und stellen zellbiologische Standardprozeduren dar (vgl. z.B. Lindl, T.; Bauer, J., Zell- und Gewebekultur, G. Fischer Verlag, Stuttgart/New York, 2. Auflage, 1989).

Gemäß einer bevorzugten Ausführungsform der Erfindung werden die permanenten Zellinien myeloiden Ursprungs von Zellen aus der monozytären Reihe oder deren Vorläufern abgeleitet, insbesondere von der Monozyten-Myelom-Zellinie U-937.

Mit zusätzlichen Behandlungen durch chemische, thermische, mechanische, zellbiologische, mikrobiologische und/oder licht-vermittelte Mittel können die in den beschriebenen Verfahren selektierten Zellen noch weiter verändert bzw. abgeleitet werden, wobei diese Verfahren auch die Einwirkung eines oder mehrerer biologischer Mediatorstoffe, wie beispielsweise Zytokine und/oder Interleukine, umfassen können.

Die auf diese Weise erhaltenen Zellinien können in vielen Fällen von noch größerem Vorteil sein, um detaillierte Informationen hinsichtlich der Rolle von Langerhans-Zellen bei immunologischen Abwehrmechanismen, z.B. bei der Untersuchung von Autoimmunerkrankungen, zu erlangen, die mit Zellinien, die das gesamte funktionelle und antigene Charakteristika von Langerhans-Zellen nicht aufweisen, nicht zugänglich sind.

Mit den erfindungsgemäßen permanenten Zellinien, vorzugsweise RAN1 und LAS1 stehen erstmals Zellinien mit einer Reinheit von nahezu 100% zur Verfügung, die die funktionellen und antigenen Charakteristika von Langerhans-Zellen aufweisen und auf einfachem Wege hergestellt werden können. Die aus dem Stand der Technik bekannten schwierigen und zumeist langwierigen Isolierungsverfahren, die in der Regel nur angereicherte, stark verunreinigte Langerhans-Zellisolate liefern, werden auf diese Weise vermieden.

Durch die im Gegensatz zu normalen Langerhans-Zellen praktisch unbegrenzte Kultivierbarkeit bzw. Vermehrbarkeit in vitro ohne Funktionsverlust lassen sich die Zellinien der vorliegenden Erfindung in besonders vorteilhafter Weise zur Durchführung von biologischen, immunologischen, medizinischen, pharmakologischen, toxikologischen, (insbesondere immuntoxikologischen) und/oder kosmetischen Untersuchungen verwenden. Die Zellinien eignen sich z.B. zur Bestimmung allergener Potentiale von Antigenen, zur Untersuchung immunologischer Zustandsbilder der Haut, zur Untersuchung der systemischen Immunität sowie von Autoimmunitätsphänomenen und Carcinogesemechanismen. Ein breites Anwendungsspektrum ermöglichen die Zellinien bei der Aufklärung und detaillierten Untersuchung bakterieller und/oder viraler Infektionskrankheiten, wobei insbesondere die Infektion mit HIV und die damit assoziierten Krankheitsbilder im Vordergrund stehen. Darüber hinaus können die erfindungsgemäßen Zellinien in bevorzugter Weise zur Untersuchung UV-vermittelter Immunsuppressionsphänomene verwendet werden.

Die erfindungsgemäßen Zellinien ermöglichen ferner erstmals die Herstellung von Test-Kits (Diagnose-Kits) für eine Vielzahl biologischer, immunologischer, medizinischer, pharmakologischer, toxikologischer und/oder kosmetischer Untersuchungen, da die Zellinien im Gegensatz zu normalen Langerhans-Zellen in Kultur stabil sind und keine Funktionsverluste aufweisen.

Die vorliegende Erfindung wird nachfolgend anhand von Beispielen näher erläutert:

### 1. Herstellung der erfindungsgemäßen permanenten Zellinien:

Das Verfahren zur Herstellung der erfindungsgemäßen permanenten Zellinien aus myeloiden Zellen, insbesondere der monocytären Reihe, ist am Beispiel des Klonierungsverfahrens für die Zellinien RAN1 und LAS1 ausgehend von einer aus peripheren Lymphknoten isolierten histiocytischen Lymphom-Zellinie nachfolgend beschrieben und in Figur 1 schematisch dargestellt.

Die histiozytische Lymphom-Zellinie U-937 ließ man zunächst einen Tag in RPMI-1640 Medium mit 10% fötalem Kälberserum (FCS) prolifierieren, bevor man nach 2, 4 bzw. 7 Tagen adhärente Zellen selektierte, die nacheinander die Passagen (vgl. z.B. Lindl, T.; Bauer, J., Zell- und Gewebekultur, G. Fischer Verlag, Stuttgart/New York, 2. Auflage, 1989) 1, 2 bzw. 4 durchlaufen hatten. Nach weiteren 7 Tagen (Passage 6) erhielt man 12% CD1-Zellen, die mit der alkalische Phosphatase anti-alkalische Phosphatase (APAAP) Färbetechnik identifiziert wurden. Nach Durchlaufen der Passage 8 (für 7 Tage) erhielt man die ersten Klone, die eine flache Morphologie aufwiesen. Die Selektion adhärenter Zellen erfolgte am 35. Tag, nachdem die Zellen Passage 10, d.h. eine erste limitierende Verdünnungsselektion, durchlaufen hatten. Nach weiteren 7 Tagen (Passage 12) wurden diejenigen Kavitäten ausgewählt, die nur adhärente Zellen enthielten, wobei 62% CD1-Zellen isoliert werden konnten. Der zweite Klonierungsschritt (zweite limitierende Verdünnunsselektion adhärenter Zellen, d.h. Passage 14) wurde wiederum über einen Zeitraum von 7 Tagen durchgeführt, und die Selektion von Kavitäten mit flachen Klonen adhärenter Zellen (Passage 16) erfolgte am 56. Tag des Verfahrens. In Passage 18 wurden am 63. Tag des Selektionsverfahrens im Hinblick auf adhärente Zellen unter Verwendung der APAAP-Färbetechnik in einem Fluoreszenz-aktivierten Zellsortierer (FACS) 92% CD1-Zellen erhalten, die nach Passage 20 am 70. Tag zur Isolierung der permanenten Zellinien RAN1 und LAS1 führten.

### 2. Untersuchung von allergenen Substanzen unter Verwendung der Zellinien RAN1 und LAS1:

Im folgenden Beispiel wird die Verwendung der Zellinien LAS1 (DSM ACC2139) und RAN1 (DSM ACC2140) zur Untersuchung von allergenen Potentialen mit Substanzen mit Hilfe der Reverse-Transkriptase-Polymerase-Chain-Reaction (RT-PCR) beschrieben.

5·10⁴ RAN1- und LAS1-Zellen pro Loch wurden für 14 h in RPMI 1640/5% Human AB-Serum bei 37°C im CO₂-Inkubator kultiviert.

Anschließend inkubierte man die Zellen mit einem Standard-Allergen (Urushiol: 1,0; 2,0 µmol), einem Standard-Irritans (SDS; 0,001, 0,0001%) oder zur Kontrolle mit Medium für längstens 30 Minuten.

Sofort im Anschluß daran wurde die zytoplasmatische mRNA dieser Zellproben mittels eines "mRNA Quick-Prep"-Kits (Pharmacia) isoliert und dann mit Hilfe eines "cDNA-First-Strand"-Synthese Kits (Pharmacia) in einzelsträngige cDNA umgeschrieben.

Diese einzelsträngige cDNA diente dann als Ausgangsmaterial für die anschließende PCR-Analytik, wobei die PCR-Reaktion nach dem Firmen-Protokoll von Perkin-Elmer unter Zuhilfenahme eines Original "Gene Amp PCR Reagent Kit" und eines "Gene Amp PCR System 9600" (Perkin Elmer) durchgeführt wurde. Die eingesetzten Primer hatten die Sequenzen:
IL-1β 5'primer:
5'ATGGCAGAAGTACCTAAGCTCGC3' (SEQ ID NO: 1)
IL-1β 3'primer:
5'ACACAAATTGCATGGTGAAGTCAGTT3' (SEQ ID NO:2)
Die amplifizierten PCR-Produkte wurden gelelektrophoretisch analysiert. Dabei führte nur das Standard-Allergen Urushiol, nicht jedoch SDS oder die Medium-Kontrolle, zu einer signifikanten Zunahme an IL-1β spezifischer m-RNA. Dieser Befund steht in Übereinstimmung mit in der Literatur für Langerhans-Zellen beschriebenen Ergebnissen, die belegen, daß dieses Interleukin bei Kontakt von Langerhans-Zellen mit Kontakt-Allergenen gebildet wird.

### 3. Untersuchung von Phänomenen der systemischen Immunität:

Im folgenden Beispiel wurden die Zellen der Linien RAN1 und LAS1 zur Untersuchung von Phänomenen der systemischen Immunität eingesetzt.

Zu diesem Zweck wurden die Zellen exemplarisch mit steigenden Dosen an TNFα (100, 500, 1000 U/ml; Fa. Genzyme) im Kulturmedium (RPMI-1640 mit 10% FCS) für 24 bis 36 h bei 37°C, 5% CO₂ inkubiert.

Im Anschluß wurde die Modulation der Oberflächenantigene CD1 und HLA-DR mittels FACS-Analyse als Kriterium der Immunkompetenz dieser Zellen untersucht. Zur Analyse der Antigene wurden die monoklonalen Antikörper T6 (anti CD1 der Firma Coulter Electronics) und IOT 2 (anti HLA-DR der Firma Immunotech) verwendet und die Zellen wie folgt behandelt:

Die Zellen wurden zunächst mit PBS gewaschen und für 10 min mit 0,5 mM Ethylendiamintetraessigsäure (EDTA) in PBS bei 37°C abgelöst. Nach 3-maligem Waschen mit PBS inkubierte man die Zellen für 20 min auf Eis mit 0,1% Ziegenserum (Firma Sigma) in PBS. Nachdem die Zellen mit PBS gewaschen wurden, teilte man sie auf Eppendorf-Gefäße (1,5 ml) auf und versetzte sie mit der entsprechenden Menge an fluoreszenzmarkiertem monoklonalem Antikörper (5µl CD1-Antikörperlösung auf 1·10⁵ Zellen, 20 µl HLA-DR-Antikörperlösung auf 0,5·10⁶ Zellen) und inkubierte für 45 min bei 4°C. Anschließend wurden die Zellen mit PBS gewaschen, in je 200 µl PBS aufgenommen, und in einem FACS-Gerät (Profile II, Coulter Electronics) wurde die Fluoreszenz der Zellen gemessen.

Die Analyse der Zellen ergab, daß TNFα bei den Zellinien RAN1 und LAS1 die Expression der Antigene stimulierte, was somit eine Stimulation der Antigen-Expressionsfähigkeit der Zellen belegt.

### 4. Untersuchung der antigen-präsentierenden Fähigkeit der Zelllinien RAN1 und LAS1:

Im folgenden Beispiel wird die Antigen-präsentierende Fähigkeit der Zellinien RAN1 und LAS1 beschrieben:
RAN1- oder LAS1-Zellen wurden in RPMI 1640 Medium mit 10% FCS in 96-Loch-Mikrotiterplatten für 24 h (Einsaat 5·10³, 1·10⁴, 2·10⁴ Zellen pro Loch) bei 37°C, 5% CO₂, inkubiert. Danach wurden die Zellen mit Mitomycin C (50µg/ml, 100 µl pro Loch in PBS) bei 37°C für 30 min inkubiert, um ihre Replikation zu blockieren.

Nach 3-maligem Waschen mit PBS wurden die Zellen mit steigenden Konzentrationen des Antigens SEB (Staphylococcus Enterotoxin B) (0 bis 100 ng/ml) beladen.

Zu diesem Ansatz wurden 2,5·10⁴, 5·10⁴ und 1·10⁵ aufgereinigte T-Lymphozyten eines gesunden humanen Spenders pro Loch zutitriert und dieser Ansatz für 3-4 Tage bei 37°C in Wasserdampf-gesättigter Atmosphäre eines CO₂-Inkubators kultiviert.

Während der letzten 6-8 h der Kultivierung wurden diese Kulturen mit 0,4 µCi ³H-Thymidin pro Loch gepulst. Im Anschluß daran erntete man die Zellen mittels eines Zellenerntegerätes auf Glasfibermatten ab, und die eingebaute Radioaktivität wurde als Maß für die T-Lymphozyten-Proliferation mittels eines Flüssigkeitsszintillationszählers bestimmt.

Sowohl RAN1 als auch LAS1 waren in der Lage, in Anwesenheit von SEB die eingesetzten T-Lymphozyten zu aktivieren. Beide Zellinien sind somit in der Lage, genau wie Langerhans-Zellen, Antigene zu präsentieren.

### 5. Untersuchungen UV-vermittelter Phänomene der Immunsuppression in der UV-B-mixed-lymphocyte reaction:

Im folgenden wurde die Eignung der Zellinien RAN1 und LAS1 für Untersuchungen UV-vermittelter Phänomene der Immunsuppression in der UV-B-Mixed-Lymphocyte-Reaction getestet.

Zu diesem Zweck wurden die Zellinien mit UV-B-Dosen (Philips TL20W/12) von 60, 120, 240 mJ/cm² bestrahlt, anschließend mit Mitomycin C (50µg/ml) behandelt, dreimal mit PBS gewaschen und anschließend mit einem Zelltiter von 5·10⁴ Zellen pro Mikrotiter-Loch in RPMI-1640 Medium verteilt. Entsprechende Kontroll-Zellen wurden vor der Mitomycin C Behandlung nicht UV-bestrahlt.

Unmittelbar danach wurden 3·10⁵ PBMNCs (Peripheral Blood Mononuclear Cells) eines gesunden humanen Donors pro Loch titriert und diese "Mixed-Lymphocyte-Reaction" für 5 Tage in RPMI-1640/10% FCS bei 37°C im CO₂-Inkubator kultiviert.

Für die letzten 6 bis 8 h der Kulturdauer wurden die Zellen mit 0,4 µCi ³H-Thymidin pro Loch gepulst und die radioaktive Einbaurate mittels eines Flüssigkeitsszintillationszählers bestimmt.

Bei applizierten UV-B-Dosen von 60, 120 und 240 mJ/cm² war die immunologische Reaktionsfähigkeit dieser Kulturansätze zu nahezu 100% supprimiert. Dieses Ergebnis belegt die Eignung der Zelllinien zur Untersuchung UV-B-induzierter Immunsuppression (analog zu Langerhans-Zellen).

### 6. Infektion der Zellinien RAN1 und LAS1 durch HIV:

Bei der nachfolgenden Untersuchung wurden die Zellen der beiden Zellinien RAN1 und LAS1 zu einem Pellet zentrifugiert und die Überstände entfernt. Überstände von der HTLV-IIIb (HIV) infizierten Zellinie H9 (ATCC CRL 8543, US-A 4 520 113) in den Verdünnungen 1:1, 1:10, 1:100 und 1:1000 wurden über Nacht mit den Zellinien RAN1 und LAS1 inkubiert, dreimal mit PBS gewaschen und in Kultur gesetzt (RPMI-1640/10% FCS).

Am 3., 5. und 7. Tag wurden Zellen zur Färbung mit einem monoklonalen Antikörper gegen das Virus-spezifische Antigen P24 bei Verwendung der alkalischen Phosphatase anti-alkalischen Phosphatase (APAAP) Färbetechnik herausgenommen.

Die Zellen waren am 5. und 7. Tag eindeutig positiv. Zusätzlich dazu wurden Überstände der in Kultur gehaltenen in vitro infizierten Zelllinien auf MT 2 Zellen übertragen. MT 2 Zellen sind gegenüber HIV-Infektion sehr sensitive Zellen. Mit Überständen, die am 5. und 7. Tag den primären infizierten Zellinien in Kultur entnommen wurden, zeigten die MT 2 Zellen nach 3 Tagen eine deutlich positive Reaktion.

Eine in situ-Hybridisierung der Zellen des 5. und 7. Tages zeigten eindeutig infizierte Zellen.

Die oben genannte Untersuchung erbrachte positive Resultate sowohl für RAN1 und LAS1 als auch für Gemische der beiden Zellinien.

Mit den oben dargestellten Ergebnissen konnte somit der sichere Beweis erbracht werden, daß die erfindungsgemäßen Zellinien sich einfach mit dem HIV-Virus infizieren lassen, wodurch damit die verschiedensten Anwendungsmöglichkeiten im Rahmen der HIV-Forschung, z.B. zur Erlangung basaler Kenntnisse, für Testkits oder zur Entwicklung von Vakzinen, Medikamenten usw., eröffnet werden.

SEQUENZPROTOKOLL

(1) ALLGEMEINE INFORMATION:

(i) ANMELDER:
(A) NAME: Beiersdorf AG
(B) STRASSE: Unnastr. 48
(C) ORT: Hamburg
(E) LAND: Bundesrepublik Deutschland
(F) POSTLEITZAHL: 20245

(ii) ANMELDETITEL: Humane Zellinien mit
Langerhans-Zell-Charakteristik

(iii) ANZAHL DER SEQUENZEN: 2

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 23 Basenpaare
(B) ART: Nukleinsäure
(C) STRANGFORM: Einzel
(D) TOPOLOGIE: linear

(ix) MERKMALE:
(A) NAME/SCHLÜSSEL: misc_feature
(B) LAGE: 1..23
(D) SONSTIGE ANGABEN: /note= "IL-1beta 5' primer;
PCR-Primer"

(xi) SEQUENZBESCHREIBUNG: SEQ ID NO: 1:

ATGGCAGAAG TACCTAAGCT CGC 23

(2) INFORMATION ZU SEQ ID NO: 2:

(i) SEQUENZ CHARAKTERISTIKA:
(A) LÄNGE: 26 Basenpaare
(B) ART: Nukleinsäure
(C) STRANGFORM: Einzel
(D) TOPOLOGIE: linear

(ix) MERKMALE:
(A) NAME/SCHLÜSSEL: misc_feature
(B) LAGE: 1..26
(D) SONSTIGE ANGABEN: /note= "IL-1beta 3' primer;
PCR-Primer"

(xi) SEQUENZBESCHREIBUNG: SEQ ID NO: 2:

ACACAAATTG CATGGTGAAG TCAGTT 26

| Tag | Verfahrensschritt |
|---|---|
| 0 | Histiocytische Zellinie aus peripheren Lymphknoten |
| 1 | Proliferation |
| 3 | Selektion adhärenter Zellen: Passage 1 |
| 7 | Selektion adhärenter Zellen: Passage 2 |
| 14 | Selektion adhärenter Zellen: Passage 4 |
| 21 | Selektion adhärenter Zellen: Passage 6, 12% CD1 Zellen (APAAP) |
| 28 | Selektion adhärenter Zellen: Passage 8, erste Klone; flache Morphologie |
| 35 | Selektion adhärenter Zellen: Passage 10, erste limitierende Verdünnungsselektion |
| 42 | Selektion von ausschließlich adhärente Zellen enthaltenden Kavitäten: Passage 12, 62% CD1 Zellen |
| 49 | Zweite limitierende Verdünnungsselektion adhärenter Zellen: Passage 14 |
| 56 | Selektion von flache Klone adhärenter Zellen enthaltenden Kavitäten: Passage 16 |
| 63 | Selektion adhärenter Zellen: Passage 18: 92% CD1 Zellen (FACS, APAAP) |
| 70 | Selektion adhärenter Zellen: Passage 20, RAN1 Zellinie, LAS1 Zellinie |

**Figur 1:** Klonierungsverfahren für die Zellinien RAN1 und LAS1
- verwendetes Medium: RPMI-1640 mit 10% fötalem Kalberserum (FCS)
- FACS: Fluoreszenz-aktivierter Zellsortierer
- APAAP: alkalische Phosphatase anti-alkalische Phosphatase Färbetechnik

## Patentansprüche

1. Permanente Zellinie myeloiden Ursprungs, die funktionelle und antigene Eigenschaften von Langerhans-Zellen aufweist.

2. Zellinie nach Anspruch 1, dadurch gekennzeichnet, daß sie von Monocyten oder deren Vorläufern abgeleitet ist.

3. Zellinie nach einem der Ansprüche 1 oder 2, dadurch gekennzeichnet, daß sie humanen Ursprungs ist.

4. Zellinie nach einem der Ansprüche 1 bis 3, dadurch gekennzeichnet, daß es sich um die Zellinie LAS1 (Hinterlegungsnummer DSM ACC2139) oder eine davon abgeleitete Zellinie handelt.

5. Zellinie nach einem der Ansprüche 1 bis 3, dadurch gekennzeichnet, daß es sich um die Zellinie RAN1 (Hinterlegungsnummer DSM ACC2140) oder eine davon abgeleitete Zellinie handelt.

6. Zellinie nach einem der Ansprüche 4 oder 5, erhältlich durch chemische, thermische, mechanische, zellbiologische, mikrobiologische und/oder licht-vermittelte Behandlung der Zellinien LAS1 (Hinterlegungsnummer DSM ACC2139) oder RAN1 (Hinterlegungsnummer DSM ACC2140).

7. Zellinie nach einem der Ansprüche 1 bis 6, dadurch gekennzeichnet, daß sie eines oder mehrere der Oberflächenantigene CD1a, HLA-DR, CD11a und CD23 exprimiert.

8. Zellinie nach einem der Ansprüche 1 bis 7, dadurch gekennzeichnet, daß ihre Fähigkeit zur Antigen-Präsentation durch UV-B-Licht unterdrückbar ist.

9. Verfahren zur Herstellung der Zellinie nach einem der Ansprüche 1 bis 8, dadurch gekennzeichnet, daß man
a) Zellen einer myeloiden Linie auswählt,
b) diese Zellen proliferieren läßt,
c) an Plastik adhärente Zellen selektiert,
d) eine erste limitierende Verdünnungsselektion durchführt,
e) an Plastik adhärente Zellen selektiert,
f) eine zweite limitierende Verdünnungsselektion durchführt,
g) an Plastik adhärente Zellen selektiert.

10. Verfahren nach Anspruch 9, dadurch gekennzeichnet, daß man in Stufe c) nacheinander die Passagen 1, 2, 4, 6, und 8 durchführt, wobei man in Passage 6 CD1⁺ Zellen und in Passage 8 Klone mit flacher Morphologie selektiert, in Stufe d) Passage 10 und in Stufe e) Passage 12 durchführt, wobei man in Passage 12 CD1⁺ Zellen selektiert, in Stufe f) Passage 14 und in Stufe g) nacheinander die Passagen 16, 18 und 20 durchführt, wobei man in Passage 16 flache Klone und in Passage 18 CD1⁺ Zellen selektiert.

11. Verfahren nach Anspruch 9 oder 10, dadurch gekennzeichnet, daß man in Stufe a) Zellen aus der monozytären Reihe oder deren Vorläufer ausgewählt.

12. Verfahren nach einem der Ansprüche 9 bis 11, dadurch gekennzeichnet, daß man die selektierten Zellen aus Stufe g) zusätzlich mit chemischen, thermischen, mechanischen, zellbiologischen, mikrobiologischen und/oder licht-vermittelten Mitteln behandelt.

13. Verfahren nach Anspruch 12, dadurch gekennzeichnet, daß man die selektierten Zellen aus Stufe g) mit einem oder mehreren biologischen Mediatorstoffe behandelt.

14. Verfahren nach Anspruch 13, dadurch gekennzeichnet, daß der Mediatorstoff ein Zytokin und/oder ein Interleukin ist.

15. Verwendung der Zellinien nach einem der Ansprüche 1 bis 8 zur Durchführung von biologischen, immunologischen, medizinischen, pharmakologischen, toxikologischen und/oder kosmetischen Untersuchungen.

16. Verwendung nach Anspruch 15 für immuntoxikologische Untersuchungen.

17. Verwendung nach einem der Ansprüche 15 oder 16 zur Bestimmung allergener Potentiale.

18. Verwendung nach einem der Ansprüche 15 bis 17 zur Untersuchung immunologischer Zustandsbilder der Haut.

19. Verwendung nach einem der Ansprüche 15 bis 18 zur Untersuchung der systemischen Immunität.

20. Verwendung nach einem der Ansprüche 15 bis 19 zur Untersuchung von Autoimmunitätsphänomenen.

21. Verwendung nach einem der Ansprüche 15 bis 20 zur Untersuchung von Carcinogenesemechanismen.

22. Verwendung nach einem der Ansprüche 15 bis 21 zur Untersuchung von bakteriellen und/oder viralen Infektionskrankheiten.

23. Verwendung nach Anspruch 22 zur Untersuchung von HIV-Infektionen.

24. Verwendung nach einem der Ansprüche 15 bis 23 zur Untersuchung UV-vermittelter Immunsuppressionsphänomene.

25. Test-Kit für biologische, immunologische, medizinische, pharmakologische, toxikologische und/oder kosmetische Untersuchungen, dadurch gekennzeichnet, daß es eine oder mehrere Zellinien nach einem der Ansprüche 1 bis 8 enthält.
